# EUROPEAN PATENT SPECIFICATION

(11) **EP 1 857 816 B1**
(45) Date of publication and mention of the grant of the patent: **10.03.2010**
(21) Application number: 06712387.7
(22) Date of filing: 26.01.2006
(51) Int. Cl.: G01N 33/15, G01N 33/50, A61K 45/00

(54) **METHOD OF SCREENING THERAPEUTIC AGENT FOR IRRITABLE BOWEL SYNDROME**
VERFAHREN ZUM SCREENING EINES THERAPEUTISCHEN WIRKSTOFFS FÜR DARMIRRITATIONSSYNDROME
PROCEDE DE CRIBLAGE D'UN AGENT THERAPEUTIQUE POUR LE SYNDROME DU COLON IRRITABLE

(30) Priority: 28.01.2005 JP 2005021151
(43) Date of publication of application: 21.11.2007
(73) Proprietor: Astellas Pharma Inc., Tokyo 103-8411 (JP)
(72) Inventor: FUNATSU, Toshiyuki, 2-chome, Chuo-ku, Tokyo, 1038411 (JP); HIRATA, Takuya, 2-chome, Chuo-ku, Tokyo, 1038411 (JP); KETO, Yoshihiro, 2-chome, Chuo-ku, Tokyo, 1038411 (JP)
(74) Representative: HOFFMANN EITLE
(86) International application number: PCT/JP2006/301205
(87) International publication number: WO 2006/080385

(56) References cited:
- WO-A-2004/062623
- AMANO M ET AL: "EFFECT OF CONDITIONED FEAR STRESS ON THE SMALL INTESTINAL PROPULSIVE ACTIVITY USEFULNESS FOR THE MODEL OF IRRITABLE BOWEL SYNDROME IBS" JAPANESE JOURNAL OF PHARMACOLOGY, JP, vol. 59, no. Suppl. 1, 1 January 1900 (1900-01-01), page 355P, XP008095856 ISSN: 0021-5198
- INOUE TAKESHI ET AL: "Effect of conditioned fear stress on serotonin metabolism in the rat brain" PHARMACOLOGY BIOCHEMISTRY AND BEHAVIOR, vol. 44, no. 2, 1993, pages 371-374, XP002494370 ISSN: 0091-3057
- LEE H J ET AL: "Amygdalar nmda receptors are critical for the expression of multiple conditioned fear responses." THE JOURNAL OF NEUROSCIENCE : THE OFFICIAL JOURNAL OF THE SOCIETY FOR NEUROSCIENCE 1 JUN 2001, vol. 21, no. 11, 1 June 2001 (2001-06-01), pages 4116-4124, XP002494371 ISSN: 1529-2401
- MERTZ HOWARD R: "Irritable bowel syndrome" NEW ENGLAND JOURNAL OF MEDICINE, MASSACHUSETTS MEDICAL SOCIETY, BOSTON, MA, US, vol. 349, no. 22, 27 November 2003 (2003-11-27), pages 2136-2146, XP008095857 ISSN: 1533-4406
- ISHIDA-TOKUDA KUMIKO ET AL: "Evaluation of perospirone (SM-9018), a novel serotonin-1 and dopamine-2 receptor antagonist, and other antipsychotics in the conditioned fear stress-induced freezing behavior model in rats" JAPANESE JOURNAL OF PHARMACOLOGY, vol. 72, no. 2, 1996, pages 119-126, XP002494372 ISSN: 0021-5198
- HASHIMOTO SHINJI ET AL: "Effects of conditioned fear stress on serotonin neurotransmission and freezing behavior in rats" EUROPEAN JOURNAL OF PHARMACOLOGY, vol. 378, no. 1, 28 July 1999 (1999-07-28), pages 23-30, XP002494373 ISSN: 0014-2999
- INOUE T ET AL: "Effects of conditioned fear stress on monoamine metabolism in the rat brain" JAPANESE JOURNAL OF PSYCHIATRY AND NEUROLOGY 1992 JP, vol. 46, no. 2, 1992, pages 574-575, XP002494374 ISSN: 0912-2036
- SAITO: 'Mansei Daicho Shincho Shigeki ni yoru IBS Byotai Model no Sakusei Oyobi CRH receptor1 (CRHER1) no Yakuwari' PHARMA MEDICA vol. 22, no. 2, 2004, pages 101 - 102, XP003000532
- HIRATA ET AL.: '5-HT3 Juyotai Kikkoyaku no Kabinsei Cho Shokogun Chiryoyaku to shiteno Yuyosei' THE CELL vol. 35, no. 10, 2003, pages 398 - 401, XP002986354
- YVETTE TACHÉ ET AL.: "Stress and the Gastrointestinal Tract: III. Stress-related alterations of gut motor function: role of brain corticotropin-releasing factor receptors" AMERICAN JOURNAL OF PHYSIOLOGY - GASTROINTESTINAL AND LIVER PHYSIOLOGY, vol. 280, 2001, pages G173-G177,

## Description

### Technical Field

This invention relates to a method of screening a therapeutic agent for irritable bowel syndrome.

### Background of the Invention

Irritable bowel syndrome (IBS) is a functional bowel disorder which does not cause an organic lesion in intestinal tissues but shows symptoms such as stomachache, abdominal inflation feeling and the like as well as abnormal bowel movement, which is classified into 3 types of a diarrhea-predominant type, a constipation-predominant type and an alternating type. The cause for IBS is complex and it is considered that abnormality of autonomic nervous system, mainly, due to the mental stress is the base and the abnormal bowel movement and stomachache caused by this further become stresses and worsen the morbid state (e.g., see Non-patent Reference 1).
Conventionally, an animal model in which abnormal evacuation is induced by a stress of restraint or by a drug has been used in the screening of a therapeutic agent for irritable bowel syndrome. As the former case, there are reports on an animal model in which the evacuation frequency was accelerated by inserting a rat under non-fasting into a restraint gage to apply stress thereto and on an animal model in which diarrhea was expressed by applying a stress to a fasting rat in the same manner. As the latter case, there are reports on an animal model in which the evacuation frequency was accelerated by administering TRH to a rat under non-fasting and on an animal model in which diarrhea was expressed by administering 5HT, PGE₂ or castor oil to a mouse (e.g., see Non-patent References 2 and 3).

On the other hand, a conditioned fear stressed animal model is known as an animal model which is used for the evaluation of antipsychotic drugs used in the treatment of depression and the like. For example, when a foot shock is applied to a rat in a shock box and the rat is again put into the same shock box 24 hours thereafter, the rat shows a freezing behavior (freezing) as a behavior related to fear or uneasiness. It has been reported that such freezing behavior is suppressed by anxiolytic drugs or sedatives such as diazepam and midazolam (e.g., see Non-patent Reference 4).
In addition, it has been reported that bowel movement is also accelerated in this conditioned fear stressed animal model in addition to the freezing behavior, and it has been reported that a depression treating agent fluoxetine suppressed both of freezing behavior and bowel movement (e.g., see Non-patent References 5, 6 and 7).
In another animal model the effect of diazepam and buspirone on the suppression of the small intestinal propulsive activity induced by conditioned fear stress was investigated. As a result, it was concluded that the conditioned fear stress induced suppression of the small intestinal propulsive activity may be a useful model of irritable bowel syndrome (e.g., see Non-patent Reference 8).

Non-patent Reference 1: Journal of Clinical Psychology, 2001, no. 62, p. 38 - 45
Non-patent Reference 2: The Journal of Pharmacology and Experimental Therapeutics, 1992, vol. 261, no. 1, p. 297 - 303
Non-patent Reference 3: Gastroenterology, 1988, vol. 94, p. 611 - 621
Non-patent Reference 4: Behavioral Neuroscience, 1988, vol. 102, no. 2, p. 233 - 243
Non-patent Reference 5: Pharmacology Biochemistry and Behavior, 1993, vol. 44, p. 371 - 374
Non-patent Reference 6: Gastroenterology, 1991, vol. 100, p. 964 - 970
Non-patent Reference 7: Brain Research, 2000, vol. 855, p. 58 - 66
Non-patent Reference 8: Japanese Journal of Pharmacology, vol. 59, Suppl. 1, 1992, p. 355P

### Disclosure of the Invention

### Problems that the Invention is to Solve

In order to create a novel therapeutic agent for irritable bowel syndrome, screening using an animal model that can efficiently evaluate a large number of test substances is essential. However, it is hard to say that the conventionally used animal models sufficiently reflect morbid state of the irritable bowel syndrome generated by a mental stress. That is, an animal model in which abnormal evacuation was induced by a stress of restraint has strong physical stimuli factors such as pressure to the abdominal part where influence on the bowel movement cannot be avoided and pain and the like, so that it is not possible to certainly say that the resulting abnormal evacuation is due to a mental stress. On the other hand, in the case of an animal model for a drug-induced abnormal evacuation, a large amount of a stress-related substance is administered exogenously, so that a possibility of generating interaction between a test substance and a biological substance cannot be excluded.
Accordingly, the present inventors have conducted extensive studies with the aim of developing a screening method which reflects morbid state of irritable bowel syndrome.

### Means for Solving the Problems

As a result, it was found that a conditioned fear stressed non-human animal model is effective in evaluating therapeutic agents for irritable bowel syndrome. In addition, it was confirmed that ramosetron hydrochloride and the like, wherein their efficacy for patients of irritable bowel syndrome has been confirmed, can improve evacuation condition and bowel movement in the conditioned fear stressed non-human animal model without exerting influence upon freezing behavior, thus resulting in the accomplishment of the present invention.
That is, the present invention relates to a method which comprises administering a test substances to a conditioned fear stressed non-human animal model, and 1) selecting a test substance which improves evacuation condition and bowel movement and also does not exert influence upon the freezing behavior, or 2) selecting a test substance which improves evacuation condition and bowel movement and also improves the freezing behavior.

### Effect of the Invention

By the present invention, therapeutic agents for irritable bowel syndrome developed by mental stress can be evaluated efficiently. The screening method of the present invention is superior from the viewpoint that abnormal evacuation based purely on a mental stress can be evaluated, in comparison with the conventional method which uses an animal model in which abnormal evacuation was induced by a restraint stress or a drug. On the other hand, this is also useful as an evaluation system for determining whether a substance whose efficacy for irritable bowel syndrome patients has been reported improves the abnormal evacuation by controlling stress-based emotional behavior or improves the abnormal evacuation by improving abnormal bowel movement. In addition, the screening method of the present invention can also achieve selective screening for a substance which can treat irritable bowel syndrome without showing antipsychotic activity and thus shows less risk of causing adverse side effects. On the other hand, it is also excellent as a method for selectively screening for a substance which can treat irritable bowel syndrome by alleviating mental stress which is considered to be the main cause for irritable bowel syndrome.

### Brief Description of the Drawings

Fig. 1 is a graph showing changes in freezing behavior and the number of evacuations after CFS loading. (Average value ± SEM of 12 cases for each group, * <0.05, ** <0.01 based on normal group (Student's t test))
Fig. 2 is a graph showing effect of drugs to be tested on freezing behavior after CFS loading. Average value ± SEM of 12 cases for each group, N: normal group, C: control group, # <0.05, ## <0.01 based on normal group (Student's t test), * <0.05 based on control group (Dunnett test)
Fig. 3 is a graph showing effect of drugs to be tested on the acceleration of proximal large intestine transport ability after CFS loading. Average value ± SEM of 13 cases for each group, N: normal group, C: control group, RAM: ramosetron hydrochloride, ALO: alosetron hydrochloride, CIL: cilansetron hydrochloride, LOP: loperamide hydrochloride, ## <0.01 based on normal group (Student's t test), ** <0.01 based on control group (Dunnett test)

### Best Mode for Carrying Out the Invention

The following describes the present invention further in detail.
The conditioned fear stressed non-human animal model to be used in the present invention is a non-human animal model to which a mental stress is applied by giving an unpleasant experience to a mammal under a certain specific condition, and again placing it under the same condition after a passage of a predetermined period of time.
Examples of the specific condition include a condition under which a mammal is put into a box or cage having such a degree of size that its body is not confined, so that it cannot escape from an unpleasant experience. Conditioning for an unpleasant experience can be strengthened by generating a sound or irradiating light for a certain period of time, though not limited thereto. In a preferred example, a mammal is put into a box, a warning sound is generated for a period of from several seconds to scores of seconds, and then an unpleasant experience of for a period of from several seconds to scores of seconds and a simultaneous irradiation of light are repeated from several times to scores of times.

Examples of the unpleasant experience include loading of a reversible stimulus to the body, such as electric shock or a shock by heat to limb.
The certain period of time between the fear conditioning and the test after administration of a test substance is not particularly limited with the proviso that it is a period of time of such a degree that the stressed state by fear conditioning is calmed down but the conditioning is not forgotten. For example, approximately from several hours to 48 hours can be cited, and approximately from 20 to 24 hours is more preferable.
Examples of the mammal include rat, mouse, Mongolian gerbil, rabbit, guinea pig, hamster and the like. Sex, week of age, body weight, the presence of delivery and the like of the mammal are not particularly limited as long as it can be employed in the intended screening.
The conditioned fear stressed non-human animal model can be produced in accordance with the conventionally known methods (e.g., Non-patent References 5, 6 and 7) or by optionally modifying the conventionally known methods.

Measurement of the evacuation condition can be carried out by the observation of the number of evacuations, weight of feces and the state of feces or the measurement of the content of moisture, though not limited thereto.
Measurement of the bowel movement can be carried out by the measurement of proximal large intestine transport ability by the marker method shown in Example 2 (Journal of Pharmacology and Experimental Therapeutics, 1993, vol. 266, p. 74 - 80), the measurement of distal large intestine transport ability by the beads discharge method shown in Example 3 (British Journal of Pharmacology, 1999, no. 128, p. 1554 - 69, 1999, no. 128, p. 1554 - 60), further by the method in which the movement is measured by arranging a sensor on the large intestine (Gastroenterology, 1991, vol. 100, p. 964 - 970) and the like, though not limited thereto.
The freezing behavior (freezing) is a state in which it does not show movement other than the movement accompanying breathing, for a predetermined period of time (e.g., from 10 to 30 seconds) or more.
The term "a test substance which improves evacuation condition and bowel movement and also does not exert influence upon the freezing behavior" means that the test substance does not shorten or prolong the time of period of freezing behavior at such a dose that it improves evacuation condition and bowel movement. In the same manner, the term "improves freezing behavior" means that the test substance shortens the time of period of freezing behavior at such a dose that it improves evacuation condition and bowel movement.

As the test substance, known or novel synthetic compounds, peptides, proteins and the like, as well as, for example, tissue extracts of warm blooded mammals, cell culture supernatants and the like, can be used.
Administration of the test substance is carried out by oral administration, intravenous administration, transdermal administration and the like, in accordance with the characteristics of the test substance, of which oral administration is most preferable. When the test substance is orally administered, a method in which it is made into a liquid state by dissolving in water or an organic solvent and forcedly administered to a mammal using a syringe, dropping pipette or the like is preferable.
The period of time after administration of the test substance until the test can be optionally adjusted based on the species of the mammal and administration method of the test substance. For example, in the case of the rats used in the following examples, about 1 hour after the administration is desirable in the case of oral administration, but in the case of intravenous administration, the test can be carried out in 5 to 30 minutes after the administration.

In carrying out the screening method of the present invention, it is desirable that a fear-unconditioned normal mammal, namely a mammal which was placed under a certain specific condition but did not give an unpleasant experience, is regarded as a normal group and used as the comparative control with the fear conditioned (CFS) group.

### Examples

The present invention is described further in detail in the following based on examples, but the invention is not limited to these examples.

### Example 1

### [Test method]

Male Sprague-Dawley rats (6 weeks of age, CLEA Japan) were used. The rats which passed 1 week or more after their receipt were divided into groups of 12 animals per group in such a manner that their average values and standard deviations of body weight became almost the same. The electric shock conditions were set in accordance with the results of preliminary examinations. That is, the rats were put into an electric shock device (17 x 17 x 39 cm, CB 2000 type, O'Hara) having an electrode set in the floor, and after 3 seconds of warning sound, their conditioning was carried out by applying an electric current of 2 mA at the maximum for 5 seconds per once per minute, a total of 15 times, and irradiating light at the time of the electric shock using 3 electric bulbs of 40 w. The control group was put into the electric shock device by the same protocol, but without applying the electric shock. From 18 to 24 hours thereafter, each drug to be tested was orally administered to each animal by force at a dose of 5 mL/kg, they were again put into the electric shock device 1 hour thereafter, and then a conditioned fear stress (CFS) loading was carried out by applying 3 seconds of warning sound and subsequent 5 seconds of light irradiation for 30 minutes.

The number of evacuations was recorded after 2, 4, 6, 8, 10, 15, 20, 25 and 30 minutes of the commencement of the CFS loading, and wet weights of feces were measured by recovering the feces at the time of the completion of the measurement. In addition, the feces were dried at 70°C for 22 hours and then their dry weights were measured to calculate the moisture content. In this connection, the evacuations just after putting them into the measuring device were excluded from the measured values of the number of evacuations and feces weights.
Regarding the measurement of freezing behavior (freezing), a case of not moving at all during 30 seconds other than the movement accompanied by breathing was evaluated as the freezing behavior. The evaluation times were set to 0, 1, 3, 5, 7, 9, 12, 13, 14, 17, 18, 19, 22, 23, 24, 27, 28 and 29 minutes after the CFS loading, and the measurement was carried out at intervals of 30 seconds per minute. The ratio (%) of showing the freezing behavior by each animal was calculated in 3 plots of from 0 to 9 minutes (Block 1), from 10 to 19 minutes (Block 2) and from 20 to 29 minutes (Block 3) after the loading.

### [Results]

### (1) Freezing behavior and evacuation increasing actions after conditioned fear stress (CFS) loading

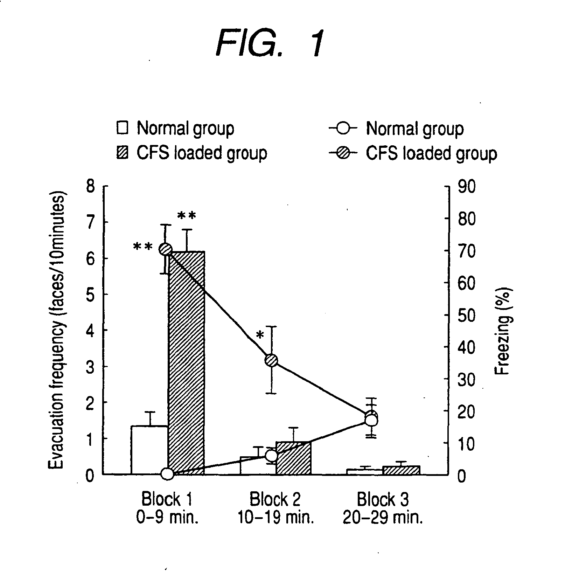

In comparison with the normal group, significant difference was not found regarding the weight gain after the CFS loading until the measurement, so that it was confirmed that invasion by the conditioning using electric shock is low. The freezing behavior as an emotional behavior when rats receive a stress was found starting just after the CFS loading, and among the 30 minutes of measuring time, it was significant in the plots of from 0 to 9 minutes (Block 1) and from 10 to 19 minutes (Block 2) in comparison with the normal group (Fig. 1). In addition, significant increase in the number of evacuations was found in 0 to 10 minutes after the CFS loading, a period when strong freezing behavior can be found, in comparison with the normal group.

### (2) Effect of therapeutic agent for irritable bowel syndrome on abnormal evacuation

In order to examine whether or not the substances whose therapeutic efficacy on irritable bowel syndrome patients have been reported also show efficacy on the abnormal evacuation found by this evaluation system, effects of 5-HT₃ receptor antagonists ramosetron hydrochloride, alosetron hydrochloride and cilansetron hydrochloride and an antidiarrheal drug loperamide hydrochloride were examined. These drugs to be tested dose-dependently lowered the wet feces weights increased by the conditioned fear stress (CFS) loading, and ED₅₀ values of the controlling ratio was as shown in Table 1. The ED₅₀ value was calculated by the linear regression (n = 12).

**[Table 1]**

| Drugs to be tested | ED₅₀ value (mg/kg) |
|---|---|
| Ramosetron hydrochloride | 0.012 |
| Alosetron hydrochloride | 0.078 |
| Cilansetron hydrochloride | 0.094 |
| Loperamide hydrochloride | 9.000 |

### (3) Effect of therapeutic agent irritable bowel syndrome on freezing behavior

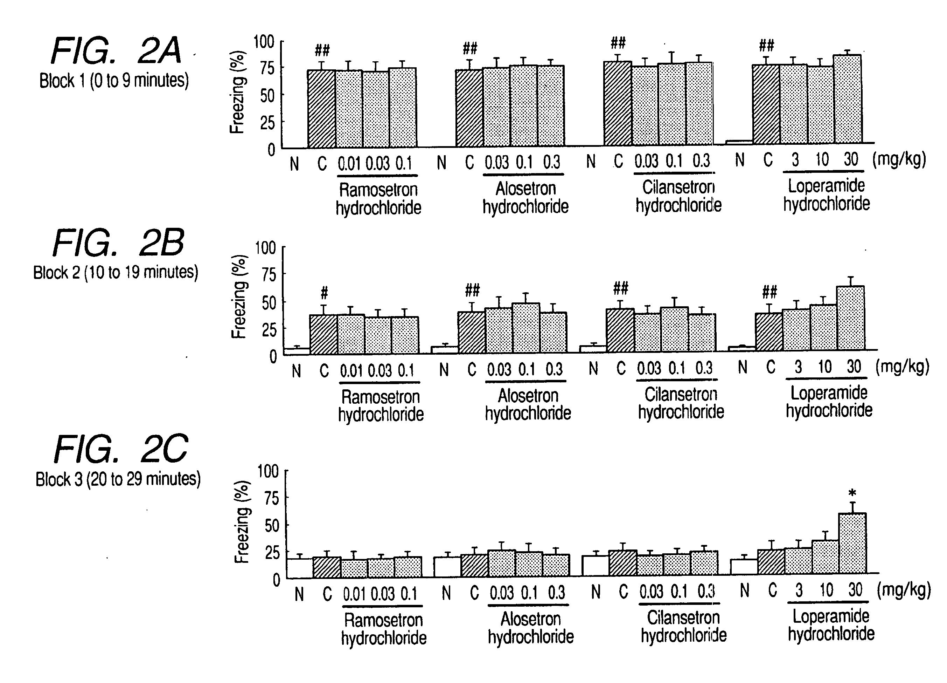

During the observation period of 30 minutes after the CFS loading, all of the drugs tested, ramosetron hydrochloride, alosetron hydrochloride and cilansetron hydrochloride, did not exert influence upon the conditioned fear stress (CFS)-induced freezing behavior at all of the doses tested, so that it was confirmed that these substances do not exert influence upon stress reactions (Fig. 2). On the other hand, loperamide hydrochloride dose-dependently prolonged the freezing behavior-expressing time of 20 to 29 minutes after CFS loading (Block 3), and its action was significant at a dose of 30 mg/kg (Fig. 2). From this result, it is considered that loperamide hydrochloride an action other than its abnormal evacuation improving action.

### Example 2

### [Test method]

Male Sprague-Dawley rats (6 to 7 weeks of age, CLEA Japan) were used. The rats which passed about 1 week after their receipt were used in the test. The electric shock conditions were set in accordance with Example 1. That is, the rats were put into an electric shock device (17 x 17 x 39 cm, CB 2000 type, O'Hara) having an electrode set in the floor, and after 3 seconds of warning sound, their conditioning was carried out by applying an electric current of 2.5 mA for 5 seconds per minute per once, a total of 15 times, and irradiating light at the time of the electric shock using 3 electric bulbs of 40 w. The normal group was put into the electric shock device by the same protocol, but without applying the electric shock. From 20 to 24 hours thereafter, each drug to be tested was orally administered to each animal by force at a dose of 5 mL/kg, they were again put into the electric shock device 1 hour thereafter, and then a conditioned fear stress (CFS) loading was carried out by applying 3 seconds of warning sound and subsequent 5 seconds of light irradiation for 30 minutes.

Measurement of proximal large intestine transport ability by a marker method was carried out by modifying the method of Kadowaki et al. (Journal of Pharmacology and Experimental Therapeutics, 1993, vol. 266, p. 74 - 80). That is, abdominal part of each rat was subjected to median incision (about 1 cm) under pentobarbital (50 mg/kg ip) anesthesia, and the ileocecal junction was extracted. A large intestinal part of about 1 cm from the connecting part was extracted from the neck by inserting canula (SP 50). The animals were individually reared for 5 days until the test, under conditions of free ingestion and free drinking. A Carmine Red solution (0.2 mL of 0.5% methyl cellulose solution containing 3 g of Carmine Red and 5 g of acacia) was injected trough the canula, CFS was loaded for 30 minutes by the aforementioned method, and then the animals were sacrificed by a decapitation device. By quickly carrying out median incision, large intestine starting part-anus part was extracted, and the whole length and marker shifting distance were measured using the canula insertion part as the starting point. The large intestine transport ability (%) was calculated as (marker shifting distance) ö (whole length) x 100.

### [Results]

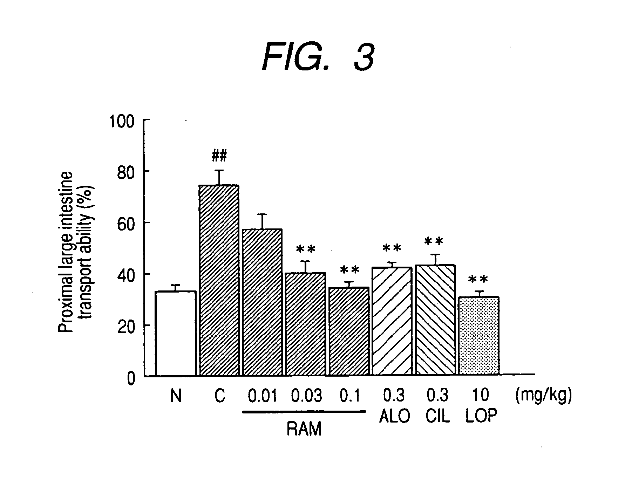

By injecting the Carmine Red solution into the large intestine starting part, the proximal large intestine transport ability 30 minutes after CFS loading was measured. Since the proximal large intestine transport ability of the CFS group was significantly increased in comparison with the normal group, it was suggested that the abnormal evacuation by this evaluation system is based on the abnormal acceleration of bowel movement, by conforming to the morbid state (Fig. 3). In addition, the ramosetron hydrochloride (0.1 mg/kg), alosetron hydrochloride (0.3 mg/kg), cilansetron hydrochloride (0.3 mg/kg) and loperamide hydrochloride (10 mg/kg), which significantly suppressed CFS-induced abnormal evacuation, lowered the proximal large intestine transport rate increased by the CFS loading.

### Example 3

### [Test method]

Male Sprague-Dawley rats (6 to 7 weeks of age, CLEA Japan) were used. The rats which passed about 1 week after their receipt were used in the test by 12 animals per group. The electric shock conditions were set in accordance with Example 1. That is, the rats were put into an electric shock device (17 x 17 x 39 cm, CB 2000 type, O'Hara) having an electrode set in the floor, and after 3 seconds of warning sound, their conditioning was carried out by applying an electric current of 2.5 mA for 5 seconds per minute per once, a total of 15 times, and irradiating light at the time of the electric shock using 3 electric bulbs of 40 w. The normal group was put into the electric shock device by the same protocol, but without applying the electric shock. From 20 to 24 hours thereafter, each drug to be tested was orally administered to each animal by force at a dose of 5 mL/kg, they were again put into the electric shock device 1 hour thereafter, and then a conditioned fear stress (CFS) loading was carried out by applying 3 seconds of warning sound and subsequent 5 seconds of light irradiation for 30 minutes.

Measurement of large intestine transport ability by a beads discharge method was carried out by modifying the method of Negri et al. (British Journal of Pharmacology, 1999, no. 128, p. 1554 - 60). That is, under light ether anesthesia, silicon beads (3 mm in diameter) were inserted in a length of 3 cm into the rectum side from the anus part using a silicon rod. After waking, the silicon rod was immediately removed to carry out CFS loading. The CFS loading starting time and silicon beads discharging time were recorded, and the measurement finishing time was set to 90 minutes in CFS group and 8 hours in normal group after the starting of the measurement. The distal large intestine transport ability (mm/min) was calculated as 30 mm ö required beads discharge time (min).

### [Results]

The results are shown in Table 2. When the conditioned fear stress (CFS) was loaded, the distal large intestine transport rate was increased in comparison with the normal group. In addition, ramosetron hydrochloride (0.1 mg/kg), alosetron hydrochloride (0.3 mg/kg), cilansetron hydrochloride (0.3 mg/kg) and loperamide hydrochloride (10 mg/kg) lowered the distal large intestine transport rate which was increased by the CFS loading.

**[Table 2]**

| Administration groups | Dose (mg/kg) | Distal large intestine transport ability (mm/min) |
|---|---|---|
| Normal group | - | 0.38 |
| Control group | - | 13.70 # |
| Ramosetron hydrochloride | 0.1 | 4.93 ** |
| Alosetron hydrochloride | 0.3 | 5.04 * |
| Cilansetron hydrochloride | 0.3 | 5.06 ** |
| Loperamide hydrochloride | 10 | 3.64 ** |

| | | |
|---|---|---|
| # P< 0.01 based on normal group (Student's t test) * P <0.05, ** P < 0.01 based on control group (Steel test) | | |

### Industrial Applicability

By the screening method of the present invention, therapeutic agents for irritable bowel syndrome developed by mental stress can be evaluated efficiently.

## Claims

1. A method of screening a therapeutic agent for irritable bowel syndrome, which comprises administering a test substance to a conditioned fear stressed non-human animal model, and measuring an evacuation condition, **characterized in that** it further comprises measuring freezing behaviour and selecting a test substance which improves evacuation condition and bowel movement and does not exert influence upon freezing behavior.

2. A method of screening a therapeutic agent for irritable bowel syndrome, which comprises administering a test substance to a conditioned fear stressed non-human animal model, and measuring an evacuation condition, **characterized in that** it further comprises measuring freezing behaviour and selecting a test substance which improves evacuation condition and bowel movement and also improves freezing behavior.

## Patentansprüche

1. Verfahren zum Screening eines therapeutischen Mittels gegen das Reizdarmsyndrom, das die Verabreichung einer Testsubstanz an einem mit einer konditionierten Furcht belasteten nicht-menschlichen Tiermodell und die Messung eines Entleerungszustands umfasst, **dadurch gekennzeichnet, dass** es ferner die Messung des Erstarrungsverhaltens und das Auswählen einer Testsubstanz umfasst, die den Entleerungszustand und den Stuhlgang verbessert und keinen Einfluss auf das Erstarrungsverhalten ausübt.

2. Verfahren zum Screening eines therapeutischen Mittels gegen Reizdarmsyndrom, das die Verabreichung einer Testsubstanz an einem mit einer konditionierten Furcht belasteten nicht-menschlichen Tiermodell und die Messung eines Entleerungszustands umfasst, **dadurch gekennzeichnet, dass** es ferner die Messung des Erstarrungsverhaltens und das Auswählen einer Testsubstanz umfasst, die den Entleerungszustand und den Stuhlgang verbessert und außerdem das Erstarrungsverhalten verbessert.

## Revendications

1. Procédé de dépistage d'un agent thérapeutique pour le syndrome du côlon irritable, lequel comprend l'administration d'une substance à tester à un modèle d'animal non humain conditionné, stressé par la peur, et la mesure d'un état d'exonération, **caractérisé en ce qu'**il comprend en outre la mesure du comportement de figeage et la sélection d'une substance à tester qui améliore l'état d'exonération et le mouvement du côlon et n'exerce pas d'influence sur le comportement de figeage.

2. Procédé de dépistage d'un agent thérapeutique pour le syndrome du côlon irritable, lequel comprend l'administration d'une substance à tester à un modèle d'animal non humain conditionné, stressé par la peur et la mesure d'un état d'exonération, **caractérisé en ce qu'**il comprend en outre la mesure du comportement de figeage et la sélection d'une substance à tester qui améliore l'état d'exonération et le mouvement du côlon et améliore également le comportement de figeage.
